# EUROPEAN PATENT APPLICATION

(11) **EP 4 563 689 A1**
(43) Date of publication of application: **04.06.2025**
(21) Application number: 23212480.0
(22) Date of filing: 28.11.2023
(51) Int. Cl.: C12M 1/107, C12M 1/08, C12M 1/34

(54) **A BIOREACTOR AND A METHOD FOR PRODUCING BIOGAS**

(71) Applicant: BioVille Oy, 83720 Kuorevaara (FI)
(72) Inventor: MÄKI, Erik, CH-8252 Schlatt (CH)
(74) Representative: Berggren Oy

(57) **Abstract**

The bioreactor comprises a tank (10) for receiving biomass, said tank having a bottom (14), a side wall (16), an upper edge (17) bordering an opening (18), a substantially gas-tight deck (12) covering said opening and means for exhausting produced biogas from the bioreactor. The bioreactor further comprises means for producing and maintaining an operating pressure inside the bioreactor, which operating pressure is lower than the atmospheric pressure. The means for exhausting produced biogas from the bioreactor may act as means for producing and maintaining an operating pressure inside the bioreactor.

## Description

### Technical field

This invention relates to a bioreactor and a method for producing biogas from organic material.

### Background Art

Methanogenesis or biomethanation is a process, where organic material is microbially converted to methane (CH₄) as the final product of metabolism. Methanogenesis takes place under anaerobic conditions and is carried out by methanogenic microbes, methanogens. In anoxic environments methanogenesis is the final step in the decomposition of biomass. The biological methanation is the main industrial process for producing biogas from organic material.

Methanogens are a diverse archaeal group of microorganisms belonging to the domain Archaea. Methanogens are strictly anaerobic and live in the neutral or weakly alkaline environments with suitable temperatures. Their population growth is affected by factors including pH, temperature, water activity, and net metabolite fluxes.

Anaerobic refers to microorganisms that are able to live without molecular oxygen and to their metabolism, where ATP (Adenosine triphosphate) is produced without oxygen or in the absence of oxygen. Anoxic is used to describe environments without molecular oxygen. Fermentation is a chemical process by which molecules are broken down anaerobically.

Anaerobic fermentation comprises four major steps: hydrolysis, acidogenesis, acetogenesis and methanogenesis. First, in hydrolysis complex organic polymers are broken down into monomers. Second, in acidogenesis the products of hydrolysis are degraded, which results in the formation of hydrogen (H₂) and carbon dioxide (CO₂) as well as non-gaseous fermentation products, i.e., low-molecular weight organic acids and alcohols. Third, in acetogenesis the non-gaseous products are further oxidized to hydrogen, CO₂ and acetate. Fourth, acetate is a direct substrate for methanogenesis, which results in the production of methane and CO₂.

Biogas production from biomass can be practiced under wet and dry fermentation conditions. Wet fermentation systems utilize organic material with a consistency of low (2 - 15 %) total solids content and high (85 - 98 %) total moisture content. In contrast, dry fermentation systems utilize organic material with consistency of high (25 - 45 %) total solids content and low (55 - 75 %) total moisture content.

Documents US 8283159 B2 and EP 1088885 A2 disclose conventional biogas reactors for producing biogas from wet organic material.

Conventional biogas reactors are large in size and the production of biogas is inefficient in relation to the amount of biomass used.

An object of the invention is to provide a bioreactor and a method for producing biogas, with which drawbacks relating to the prior art can be diminished.

The object of the invention is achieved with the bioreactor and method, which are characterized in what is disclosed in the independent patent claims. Some preferred embodiments of the invention are disclosed in the dependent claims.

### Summary of the invention

The bioreactor according to the invention comprises a tank for receiving biomass, said tank having a bottom, a side wall, an upper edge bordering an opening, a substantially gas-tight deck covering said opening and means for exhausting produced biogas from the bioreactor. The bioreactor further comprises means for producing and maintaining an operating pressure inside the bioreactor, which operating pressure is lower than the atmospheric pressure. Preferably, said means for exhausting produced biogas from the bioreactor comprises a gas pipe and a gas extractor configured to remove biogas from the bioreactor.

In some embodiments of the bioreactor according to the invention said means for exhausting produced biogas from the bioreactor are configured to act as means for producing and maintaining an operating pressure inside the bioreactor.

In some embodiments of the bioreactor according to the invention said side wall comprises an upper part having a shape of a cylinder and a lower part having a shape of a truncated cone.

Some embodiments of the bioreactor according to the invention further comprise means for circulating biomass inside said tank.

In some embodiments of the bioreactor according to the invention said means for circulating biomass inside the tank comprise a preferably cylindrical flow channel and a flow generator inside or near said flow channel. Preferably, said flow channel is inside said tank in a substantially vertical position and the flow channel has a lower end opening near the bottom of the tank and an upper end opening near the upper edge of the tank.

In some embodiments of the bioreactor according to the invention the flow channel has a first cross-sectional area and a part of the tank bordered by the upper part of the side wall and the flow channel has a second cross sectional area, which first and second cross sectional areas are substantially equal.

Some embodiments of the bioreactor according to the invention further comprise at least one heat exchanger configured to heat the biomass inside the tank.

The method according to the invention for producing biogas in a bioreactor according to the invention comprises steps of feeding biomass into the bioreactor, creating suitable conditions for biogas production inside the bioreactor and removing produced biogas from the bioreactor. The method further comprises steps of producing an operating pressure inside the bioreactor, which operating pressure is lower than the atmospheric pressure and maintaining said operating pressure inside the bioreactor during the production of the biogas. The operating pressure can be 0,1-0,95 bar, preferably 0,75-0,95 bar, most preferably 0,85-0,95 bar.

In some embodiments of the method according to the invention said step of maintaining said operating pressure during the production of the biogas comprises removing biogas from the bioreactor substantially at the same rate as the biogas is produced.

In some embodiments of the method according to the invention further comprise steps of circulating the biomass inside the tank by causing the biomass to flow upwards via a first section of the tank, preferably via the flow channel, and causing the biomass to flow downwards through a second section of the tank, preferably via the gap between the side wall of the tank and the flow channel. Preferably the circulated biomass is in a form of a slurry containing a substantial amount of water.

Some embodiments of the method according to the invention further comprise steps of controlling and, preferably continuously, adjusting the temperature of the biomass inside the tank during the production of the biogas to ensure adequate growing conditions for the microbes. Preferably the steps of controlling and adjusting the temperature of the biomass inside the tank comprises steps of setting a target temperature range for the biomass, continuously measuring the temperature of the biomass inside the tank and heating the biomass to reach the target temperature range, if the measured temperature deviates from the set target temperature range. The target temperature range may be 20°C-40°C to enhance microbe growth in a mesophilic process or 40°C-80°C to enhance microbe growth in a thermophilic process.

An advantage of the invention is that it enhances the production of biogas significantly compared to the prior art methods and bioreactors. The biogas production takes place faster and more efficiently than in known bioreactors, saving biomass resources and improving bioenergy production.

A further advantage of the invention is that the bioreactor is structurally simple and compact, whereby the manufacturing costs of the bioreactor are low. Simple and compact structure requires less space and makes it possible to the place the bioreactor near to urban environment enabling decentralized energy production. Efficiency of the existing conventional large and inefficient bioreactors can be improved by utilizing the invention.

Another advantage of the invention is that operation costs of the bioreactor and the production costs of the bioreactor are low.

An advantage of one preferred embodiment of the invention is that it prevents the likely sedimentation of solid biomass materials in the tank.

### Brief Description of the Drawings

In the following the invention will be described in detail, by way of examples, with reference to the accompanying drawings in which,
- Fig. 1: depicts an embodiment of the bioreactor according to the invention as a side elevation,
- Fig. 2: depicts the bioreactor shown in fig. 1 seen from above and
- Fig. 3: depicts an embodiment of the method according to the invention as a flow chart.

### Detailed description

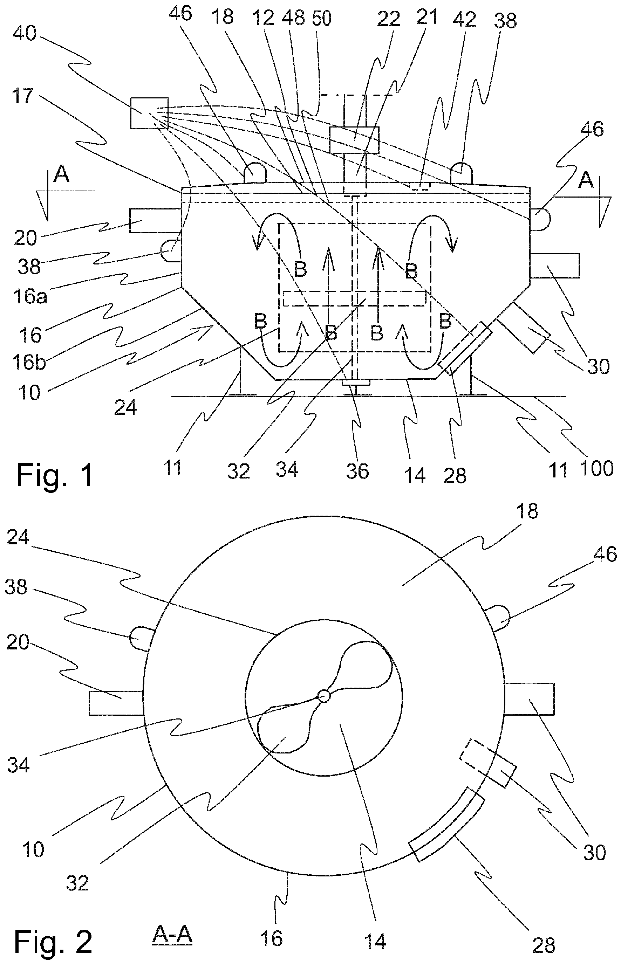

In figures 1 and 2 an example of a bioreactor according to the invention is shown. Fig. 1 depicts the bioreactor as a side elevation and fig. 2 seen from above. In the following both figures are explained simultaneously.

The bioreactor comprises a tank 10 for receiving biomass. Preferably, the biomass refers here to a biodegradable organic material containing a significant amount of water, whereby the biomass is in a form of a slurry or sludge. Therefore, the tank is substantially watertight. The tank has a side wall 16 comprising a substantially cylindrical upper part 16a and a lower part 16b having a shape of a truncated cone. At the lower edge of the lower part there is a substantially flat bottom 14. The upper part 16a of the side wall has an upper edge 17, which defines an opening 18, which is covered by an openable deck 12. The deck is a substantially rigid element, which is made of a suitable rigid material, such as reinforced plastic or metal. At the lower part of the tank there are support legs 11, on which the tank can rest on a bearing surface 100, such as ground surface or concrete slab. The tank is supported on the bearing surface in substantially vertical position as shown in fig. 1, whereby the upper edge 17 lies in an horizontal plane defining an maximum upper level for the biomass inside the tank. The tank can be made of any suitable material, such as concrete, reinforced plastic or metal, such as steel.

On the side wall 16 of the tank 10 there is at least one inlet 20, through which fresh biomass can enter inside the tank from the biomass source not shown in the figures. In the tank shown in figures 1 and 2 there is only one pipe-like inlet extending through the upper part 16a of the side wall 16. Further, on the side wall 16 of the tank 10 there is at least one outlet 30, through which used biomass can be removed from the tank. In the tank shown in figures 1 and 2 there are two pipe-like outlets; the first one extending through the upper part 16a of the side wall and the second one extending through the lower part 16b of the side wall. The inlet and the outlets are in in opposing sides of the tank.

Inside the tank there is a flow channel 24 having a substantially cylindrical wall. The flow channel is supported to the bottom and/or to the side wall of the tank into a substantially vertical position, i.e. perpendicular to the bottom, with support elements not shown in the figures. The flow channel has a lower end opening near the bottom of the tank and an upper end opening to the upper part of the tank near the level defined by the upper edge 17. Inside the flow channel there is a flow generator comprising a blade 32, supported on a shaft 34, which extends through the bottom and is rotated by a motor 36.

When the shaft and the blade connected to it are rotated, the blade forces the biomass inside the flow channel to flow upwards. The upwards flowing biomass exits the flow channel through the upper end of the flow channel, whereafter the biomass starts to flow downwards between the side wall of the tank and the outer surface of the flow channel as shown by the arrows B in fig. 1. The cross sectional area of the flow channel, here referred as a first cross-sectional area, and the cross sectional area of the part of the tank bordered by the upper part 16a of the side wall 16 and the flow channel 24, here referred as a second cross sectional area, are substantially equal areas in size, whereby the biomass flows inside the flow channel and outside the flow channel with a substantially same speed but into opposing directions.

On the side wall of the tank there is at least one heat exchanger 28 and on deck 12 and or on the side wall there is/ are at least one temperature sensor 38 configured to measure the temperature of the biomass inside the tank. The heat exchanger is used as a heating device for heating the biomass inside the tank to a higher temperature. The heat exchanger can be an electrical device or it may include a piping through which heat carrying fluid is directed to flow. The heat exchanger(s) and the temperature sensor(s) are connected to a control device 40, which is configured to operate the heat exchanger(s) based on the measured temperatures of the biomass.

The bioreactor further comprises a gas pipe 21, that extends through the deck 12 and a gas pump acting as gas extractor 22 which exhausts the produced biogas from inside the bioreactor to outside the bioreactor to a desired destination, such as a gas storage unit or to a gas delivery station. The gas pipe and the gas extractor are also used for removing air from the bioreactor and for producing and maintaining a desired operating pressure inside the bioreactor in the gas chamber 48 between the deck 12 and the upper surface 50 of the biomass. The operation pressure of the bioreactor is lower than the atmospheric pressure. On the deck there is and at least one pressure sensor 42 configured to measure the pressure in the gas chamber bordered by the upper surface of the biomass and the deck. The gas extractor and the pressure sensor(s) are connected to the control device 40 which is configured to operate the gas extractor based on the measured pressure inside the bioreactor.

The bioreactor further comprises at least one volume sensor 46 configured to measure the volume of biomass inside the tank. The volume sensor(s) may be installed on deck 12 and/or on the side wall 16 of the tank. The volume information measured by the volume sensors is used to adjust the feeding rate and removal rate of the fermented biomass to the keep the upper surface 50 of the biomass inside the tank at a desired level.

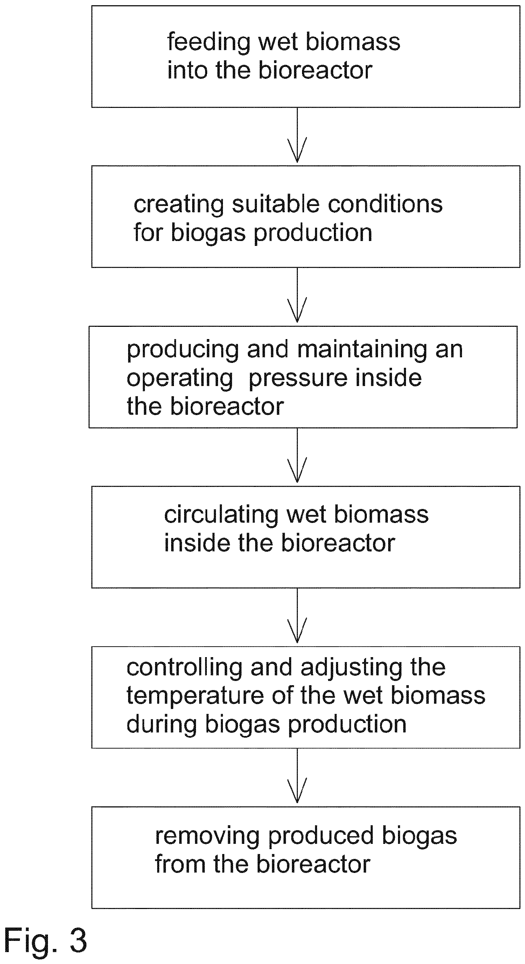

In fig 3 an embodiment of the method according to the invention is depicted as a flow chart.

The method according to the invention relates to producing biogas through methanogenesis in a bioreactor in anaerobic conditions. The produced biogas is a mixture of methane, CO₂ and small quantities of other gases, produced by anaerobic microbes. The method comprises a step of feeding biomass into the tank 10 of the bioreactor via one or more inlets 20. Preferably the biomass is in a form of a slurry containing a substantial amount of water. When the biomass is in liquid form, the feeding is preferably done by pumping the biomass from a biomass source or biomass storage with suitable pumping means. While feeding the biomass the upper surface of the biomass inside the tank is continuously monitored with volume sensors 46 to control the feeding phase and to avoid overfill. When upper surface of the biomass inside the tank reaches the desired level, feeding of the biomass is stopped. During feeding of the biomass or at least soon after the feeding the tank is closed with the deck 12, whereby a substantially hermetic gas chamber 48 is created between the upper surface 50 of the biomass and the deck 12. The desired level of the upper surface of the biomass is chosen in a way, that the hermetic gas chamber has an adequate volume and a sufficient clearance between the upper surface of the biomass and the deck during production of the biogas. Preferably the clearance between the upper surface of the biomass and the deck is at least 4-8 of the total height of the tank of the bioreactor.

Next, suitable conditions for biogas production are created inside the bioreactor. This step comprises at least removing air from the bioreactor, more precisely from the gas chamber between the deck and upper surface of the biomass to enable anaerobic growing environment for methanogenic microbes. The air is removed from the gas chamber using the same means, by which the subsequently produced biogas is exhausted from the bioreactor.

The step of creating suitable conditions for biogas production may further comprise heating the biomass to a temperature which is suitable for the methanogenic microbes. In the method the biomass can be heated to 20°C-40°C to enhance microbe growth in mesophilic process or 40°C-80°C to enhance microbe growth in thermophilic process.

In the method an operating pressure, lower than atmospheric pressure, is produced inside the bioreactor, more precisely within the bioreactor's gas chamber. The operating pressure is maintained inside gas chamber during production of the biogas. In principle, the operating pressure inside the gas chamber may be 0,1-0,95 bar. In practice the method has been proven to work nicely, when the operation pressure is slightly less than the atmospheric pressure, preferably 0,75-0,95 bar, most preferably 0,85-0,95 bar. The operating pressure is produced with the same means, by which the subsequently produced biogas is exhausted from the bioreactor.

Preferably, the method further comprises steps of circulating the biomass inside the tank by causing the biomass to flow upwards via a first section of the tank 10 and causing the biomass to flow downwards via a second section of the tank. Preferably the first section of the tank is the flow channel 24 and the second section is the gap between the side wall 16 of the tank 10 and the flow channel 24, whereby the biomass flows upwards inside the flow channel and downwards outside the flow channel. However, it is also possible to arrange for the biomass to flow downwards via the flow channel and upwards via the gap between the side wall 16 of the tank 10 and the flow channel 24. The forced circulation of the biomass inside the tank is created by rotating the blade 32 inside the flow channel by using the motor 36.

Preferably the method further comprises steps of controlling and adjusting the temperature of the biomass inside the tank 10 during production of the biogas to ensure adequate growing conditions for the microbes. The temperature is controlled preferably continuously with the temperature sensors 38.

The steps of controlling and adjusting the temperature of the biomass inside the tank 10 may comprise setting a target temperature range for the biomass, continuously measuring the temperature of the biomass inside the tank and heating the biomass with the heat exchanger(s) 28 towards target temperature range, if the measured temperature deviates from the set target temperature range.

The methanogenic microbes, i.e. the methanogens, produce biogas at 20°C-40°C by a mesophilic process and at 40°C-80°C or even higher by a thermophilic process. Thus, a suitable target temperature range can be chosen based on the prevailing ambient temperature at the biogas production site and/or based on the initial temperature of the biomass to minimize the need for the heating energy.

Finally, the method according to the invention comprises a step of removing produced biogas from the bioreactor. The biogas is removed from the gas chamber 48 via the gas pipe 21. The suction force is generated by the gas extractor 22. Preferably, the biogas is removed from the bioreactor substantially at the same rate as the "new" biogas is produced to maintain the created operating pressure during the production of the biogas. The operating pressure inside the gas chamber is continuously monitored with the pressure sensor 42. If the measured operating pressure deviates too much from the desired operating pressure, biogas is removed from the gas chamber.

The method according to the invention may run continuously, whereby fermented biomass residue is removed from the tank of the bioreactor through outlet 30 continuously or periodically and simultaneously "fresh" biomass is fed into the tank via inlet 20 to replace the removed biomass to maintain a sufficient amount of nutrients for the microbes. The amount of biomass inside the tank is controlled with volume sensor(s) 46. The control device is configured to automatically stop the biomass feeding process, when the volume sensors detect, that the upper surface of the biomass has reached the desired height level. The correct ratio between the amount of biomass residue removed from the bioreactor and the amount of biomass fed into the bioreactor over a specific period of time has been established through trial and error by monitoring gas production and comparing the gas production with the stay of the biomass inside the bioreactor.

The method according to the invention optimizes the biology and ecology of methanogens in a single-phase process or after the acetogenesis sub-process in a multi-phase process. The purpose of the invention is to enhance biogas production in various types of organic material-digestion plants or bioreactors. Methanogens require a continuous supply of nutrients, which is one of the three basic requirements for their living and reproduction, in addition to the absence of oxygen and specific temperatures. As with any living organism, methanogens require a continuous nutrient supply to maintain continuous cell division. In the method according to the invention, it is possible to significantly enhance the nutrient supply to methanogens, resulting in a multiplication of cell division and having a significant effect on biogas production.

In natural conditions, the produced biogas creates bubbles or gas pockets around the methanogens. Microbes inside the bubble or air pocket have no access to new nutrient sources, i.e. to the biomass. Microbe growth and cell division can begin again only after the gas bubbles rise to the surface of the biomass, expands and finally bursts when entering the gas chamber. In the invention the bursting of the gas bubbles in enhanced by creating an operating pressure inside the gas chamber, which operating pressure is lower than the ambient pressure.

Biogas is produced within the entire content of the biomass. The produced gas bubbles tend to rise upwards towards the surface of the biomass. In the invention the rising velocity of the gas bubbles is enhanced by causing an upwards directing flow inside flow channel, whereby the gas bubbles enter into the gas chamber earlier than in normal conditions. When the biomass exits from the flow channel it starts to flow downwards through the cap between the side wall of the tank and the flow channel. During the flow the microbes, which are no longer surrounded by a gas bubble, have access to nutrients contained in the biomass, whereby they start to grow and produce biogas.

The bioreactor according to the invention combines and optimizes the mechanical, physical and chemical properties of the process to favor methanogens. The equilibrium of the chemical reactions is enhanced so that carbon dioxide dissolved in water easily separates from the water content of the biomass. Carbon dioxide is always present in the biomass in varying amounts, depending on the specific situation and the amount of water-soluble organic material. Additionally, methanogens produce a small amount of carbon dioxide as a byproduct of their metabolism when breaking down more complex organic compounds containing carbon and oxygen atoms. Some of the carbon is released as carbon dioxide, forming gas bubbles.

When the pressure in the gas chamber of the bioreactor decreases and temperature changes, the chemical equilibrium shifts towards carbon dioxide separation. The separated carbon dioxide moves upwards in the biomass, carrying methane with it. The process conditions must remain stable, so lower operating pressure in the gas chamber must be maintained. The operating pressure is maintained by removing the biogas and carbon dioxide from the gas chamber of the bioreactor at substantially same rate as biogas and carbon dioxide are released into the gas chamber from the biomass.

Carbon dioxide separation, which causes visually detectable bubbling on the upper surface of the biomass, is very strong. Consequently, it is obvious that the formed CO₂ gas bubbles also carry the methane and other produced gases upwards towards the surface of the biomass and further into the gas chamber.

Some preferred embodiments of the bioreactor and method for producing biogas have been disclosed above. The invention is not limited to the solutions explained above, but the invention can be applied in different ways within the limits set by the patent claims.

### Reference signs:

- 10: tank
- 11: support leg
- 12: deck
- 14: bottom
- 16: side wall
- 16a: upper part
- 16b: lower part
- 17: upper edge
- 18: opening
- 20: inlet
- 21: gas pipe
- 22: gas extractor
- 24: flow channel
- 28: heat exchanger
- 30: outlet
- 32: blade
- 34: shaft
- 36: motor
- 38: temperature sensor
- 40: control device
- 42: pressure sensor
- 46: volume sensor
- 48: gas chamber
- 50: upper surface
- 100: bearing surface

## Claims

1. A bioreactor comprising a tank (10) for receiving biomass, said tank (10) having a bottom (14), a side wall (16), an upper edge (17) bordering an opening (18), a substantially gas-tight deck (12) covering said opening (18) and means for exhausting produced biogas from the bioreactor, **characterised in that** it further comprises means for producing and maintaining an operating pressure inside the bioreactor, which operating pressure is lower than the atmospheric pressure.

2. The bioreactor according to claim 1, **characterised in that** said means for exhausting produced biogas from the bioreactor comprises a gas pipe (21) and a gas extractor (22) configured to remove biogas from the bioreactor.

3. The bioreactor according to claim 1 or 2, **characterised in that** said means for exhausting produced biogas from the bioreactor are configured to act as means for producing and maintaining an operating pressure inside the bioreactor.

4. The bioreactor according to any of the claims 1 to 3, **characterised in that** said side wall (16) comprises an upper part (16a) having a shape of a cylinder and a lower part (16b) having a shape of a truncated cone.

5. The bioreactor according to any of the claims 1 to 4, **characterised in that** it further comprises means for circulating biomass inside said tank (10).

6. The bioreactor according to claim 5, **characterised in that** said means for circulating biomass inside the tank (10) comprise a preferably cylindrical flow channel (24) and a flow generator inside or near said flow channel (24).

7. The bioreactor according to claim 6, **characterised in that** said flow channel (24) is inside said tank (10) in substantially vertical position and the flow channel (24) has a lower end opening near the bottom (14) of the tank (10) and an upper end opening near the upper edge (17) of the tank (10).

8. The bioreactor according to claim 6 or 7, **characterised in that** the flow channel (24) has a first cross-sectional area, and a part of the tank (10) bordered by the upper part (16a) of the side wall (16) and the flow channel (24) has a second cross sectional area, which first and second cross-sectional areas are substantially equal.

9. The bioreactor according to any of the claims 1 to 8, **characterised in that** it further comprises at least one heat exchanger (28) configured to heat the biomass inside the tank.

10. A method for producing biogas in a bioreactor according to any of the claims 1 to 9, the method comprising steps of
- feeding biomass into the bioreactor
- creating suitable conditions for biogas production inside the bioreactor and
- removing produced biogas from the bioreactor
**characterised in that**, the method further comprises steps of
- producing an operating pressure inside the bioreactor, which operating pressure is lower than the atmospheric pressure and
- maintaining said operating pressure inside the bioreactor during the production of the biogas.

11. The method according to claim 10, **characterized in that** the operating pressure is 0,1-0,95 bar, preferably 0,75-0,95 bar, most preferably 0,85-0,95 bar.

12. The method according to claim 10 or 11, **characterized in that** said step of maintaining said operating pressure during the production of the biogas comprises removing biogas from the bioreactor substantially at the same rate as the biogas is produced.

13. The method according to any one of the claims 10 to 12, **characterized in that** the method further comprises steps of circulating the biomass inside the tank (10) by causing the biomass to flow upwards via a first section of the tank (10), preferably via the flow channel (24), and causing the biomass to flow downwards through a second section of the tank (10), preferably via the gap between the side wall (16) of the tank (10) and the flow channel (24).

14. The method according to any one of the claims 10 to 13, **characterized in that** the method further comprise steps of controlling and, preferably continuously, adjusting the temperature of the biomass inside the tank (10) during the production of the biogas to ensure adequate growing conditions for the microbes.

15. The method according to claim 14, **characterized in that** the steps of controlling and adjusting the temperature of the biomass inside the tank (10) comprise steps of
- setting a target temperature range for the biomass, preferably 20°C-40°C or 40°C-80°C,
- continuously measuring the temperature of the biomass inside the tank and
- heating the biomass to reach the target temperature range, if the measured temperature deviates from the set target temperature range.
